(19) Europäisches Patentamt
European Patent Office
Office européen des brevets

(11) Veröffentlichungsnummer: **0 119 572**
**B1**

(12) # EUROPÄISCHE PATENTSCHRIFT

(45) Veröffentlichungstag der Patentschrift: **10.08.88**

(51) Int. Cl.⁴: **C 07 D 249/08**, A 01 N 43/64

(21) Anmeldenummer: **84102657.8**

(22) Anmeldetag: **12.03.84**

(54) **Substituierte Phenethyl-triazolyl-Derivate, Verfahren zu ihrer Herstellung sowie ihre Verwendung als Fungizide.**

(30) Priorität: **24.03.83 DE 3310830**

(43) Veröffentlichungstag der Anmeldung:
**26.09.84 Patentblatt 84/39**

(45) Bekanntmachung des Hinweises auf die Patenterteilung:
**10.08.88 Patentblatt 88/32**

(84) Benannte Vertragsstaaten:
**AT BE CH DE FR GB IT LI NL SE**

(56) Entgegenhaltungen:
**EP-A-0 113 839**
**EP-A-0 146 047**
**DE-A-3 139 370**
**FR-A-2 336 129**
**FR-A-2 361 375**
**GB-A-2 023 141**

(73) Patentinhaber: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 1 (DE)**
Erfinder: **Knops, Hans- Joachim, Dr., Köpenicker Strasse 35, D-4019 Monheim (DE)**
Erfinder: **Büchel, Karl Heinz, Prof. Dr., Dabringhausener Strasse 42, D-5093 Burscheid (DE)**
Erfinder: **Reinecke, Paul, Dr., Lessingstrasse 11, D-5090 Leverkusen (DE)**

EP 0 119 572 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue substituierte Phenethyl-triazolyl-Derivate, ein Verfahren zu ihrer Herstellung sowie ihre Verwendung als Fungizide.

Es ist bereits bekannt geworden, daß substituierte 1-Benzyloximino- bzw. Benzyloxy-1-phenyl-2-triazolyl-ethane, wie beispielsweise 1-(4-Chlorbenzyl-oximino)-1-(2,4-dichlorphenyl)-2-(1,2,4-triazol-1-yl)-ethan oder 1-(2,6-Dichlorbenzyloximino)-1-(2,4-dichlorphenyl)-2-(1,2,4-triazol-1-yl)-ethan bzw. 1-(2-Chlorbenzyloxy)-1-(2,4-dichlorphenyl)-2-(1,2,4-triazol-1-yl)-ethan, gute fungizide Eigenschaften aufweisen (vergleiche DE-A-2 816 817 bzw. DE-A-2 547 953). Die Wirkung dieser Verbindungen ist jedoch, insbesondere bei niedrigen Aufwandmengen und -konzentrationen nicht immer voll befriedigend.

Ferner sind aus der FR-A-2 336 129 und der EP-A-0 113 839 bestimmte Oximether-Derivate mit fungiziden Eigenschaften bekannt. Die entsprechenden regulären Ether werden aber nicht offenbart.

Schließlich sind aus der GB-A-2 023 141 Phenethyl-imidazolyl-ether mit antimykotischer Wirksamkeit bekannt. Es werden jedoch keine Triazolyl-Derivate erwähnt.

Es wurden nun neue substituierte Phenethyl-triazolyl-Derivate der Formel

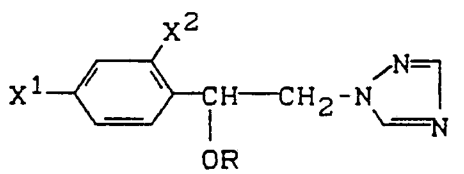

(I)

in welcher

$X^1$ und $X^2$ gleich oder verschieden sind und für Wasserstoff, Fluor, Chlor, Brom oder Halogenalkyl mit 1 oder 2 Kohlenstoffatomen und 1 bis 5 gleichen oder verschiedenen Halogenatomen stehen, wobei $X^1$ und $X^2$ aber nicht gleichzeitig Wasserstoff bedeuten,

und

R für gegebenenfalls einfach bis dreifach, gleich oder verschieden im Phenylteil durch Halogen und Alkyl mit 1 bis 4 Kohlenstoffatomen substituiertes Phenoxyethyl steht,

sowie deren Säureadditions-Salze und Metallsalz-Komplexe gefunden.

Weiterhin wurde gefunden, daß man die substiutierten Phenethyl-triazolyl-Derivate der Formel (I) sowie deren Säureadditions-Salze und Metallsalz-Komplexe erhält, wenn man Phenethyl-triazolyl-Derivate der Formel

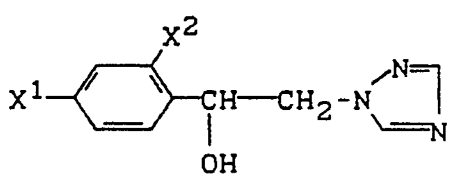

(II)

in welcher

$X^1$ und $X^2$ die oben angegebene Bedeutung haben,

mit Halogeniden der Formel

Hal-R (III)

in welcher

R die oben angegebene Bedeutung hat und

Hal für Chlor oder Brom steht,

gegebenenfalls in Gegenwart einer starken Base und in Gegenwart eines Verdünnungsmittels umsetzt, und gegebenenfalls noch an die so erhaltenen Verbindungen der Formel (I) eine Saure oder ein Metallsalz addiert.

Überraschenderweise zeigen die erfindungsgemäßen Verbindungen der Formel (I) eine bessere fungizide Wirksamkeit als die aus dem Stand der Technik bekannten substituierten 1-Benzyloximino- bzw. -Benzyloxy-1-phenyl-2-triazolyl-ethane, wie beispielsweise 1-(4-Chlorbenzyl-oximino)-1(2,4-dichlorphenyl)-2-(1,2,4-triazol-yl)--ethan und (1-(2,6-Dichlorbenzyloximino)-1-(2,4-dichlor-phenyl)-2-(1,2,4-triazol-1-yl)-ethan sowie 1-(2-Chlor-

benzyloxy)-1-(2,4-dichlorphenyl)-2-(1,2,4-triazol-1-yl)-ethan, welche konstitutionell und wirkungsmäßig naheliegende Verbindungen sind. Die erfindungsgemäßen Wirkstoffe stellen somit eine Bereicherung der Technik dar.

Bevorzugte erfindungsgemäße Stoffe sind diejenigen Verbindungen der Formel (1), in denen

$X^1$ und $X^2$ gleich oder verschieden sind und für Fluor, Chlor oder Trifluormethyl stehen; sowie für Wasserstoff, wenn der andere Substituent nicht für Wasserstoff steht,

und

R für gegebenenfalls einfach bis dreifach, gleich oder verschieden im Phenylteil durch Fluor, Chlor oder Methyl substituiertes Phenoxyethyl steht.

Bevorzugte erfindungsgemäße Verbindungen sind auch Additionsprodukte aus Säuren und denjenigen substituierten Phenethyl-triazolyl-Derivaten der Formel (I), in denen die Substituenten $X^1$, $X^2$ und R die Bedeutungen haben, die bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Phenylethyltriazolyl-Derivaten der Formel (I), in denen die Substituenten $X^1$, $X^2$ und R die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, Salpetersäure und Schwefelsäure.

Verwendet man beispielsweise 1-(2,4-Dichlorphenyl)-2-(1-2,4-triazol-1-yl)-1-ethanol und 2,4-Dichlorphenoxy- -ethylbromid als Ausgangsstoffe, so kann der Reaktionsablauf des erfindungsgemäßen Verfahrens durch das folgende Formelschema wiedergegeben werden:

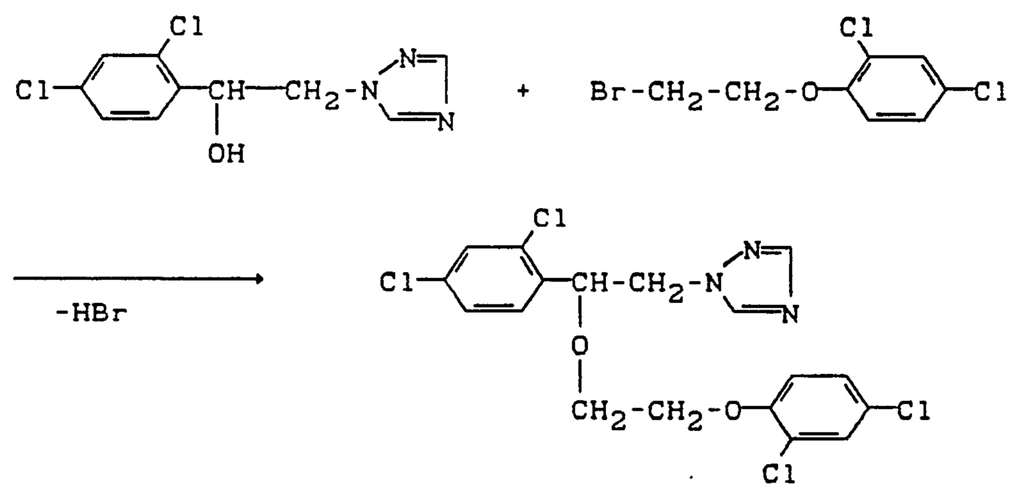


Die bei der Durchführung des erfindungsgemäßen Verfahrens als Ausgangsstoffe benötigten Phenethyl-triazolyl-Derivate sind durch die Formel (II) allgemein definiert. In dieser Formel haben $X^1$ uns $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Phenethyl-triazolyl-Derivate der Formel (II) sind bekannt (vergleiche die Deutschen Offenlegungsschriften 2 431 407, 2 547 953 und 2 816 817). Sie werden erhalten, indem man in einer ersten Stufe ω-Halogen-acetophenone mit 1,2,4-Triazol in Gegenwart eines Säurebinders bei Temperaturen zwischen 20 und 120°C umsetzt und die entstehenden ω-(1,2,4-Triazol-1-yl)-acetophenone in einer zweiten Stufe in allgemein bekannter Art und Weise mit komplexen Hydriden oder mit Aluminiumisopropylat reduziert.

Die außerdem für das erfindungsgemäße Verfahren als Ausgangsstoffe zu verwendenden Halogenide sind durch die Formel (III) allgemein definiert. In dieser Formel steht R vorzugsweise für diejenigen Reste, die bereits im Zusammenhang mit der Beschreibung der erfindungsgemäßen Stoffe der Formel (I) vorzugsweise für diesen Substituenten genannt wurden.

Die Halogenide der Formel (III) sind allgemein bekannte Verbindungen der organischen Chemie.

Für die erfindungsgemäße Umsetzung kommen als Verdünnungsmittel inerte organische Lösungsmittel infrage. Hierzu gehören vorzugsweise Ether, wie Tetrahydrofuran und Dioxan; aromatische Kohlenwasserstoffe, wie Toluol und Benzol; sowie Hexamethyl-phosphorsäure-triamid, Säureamide, wie

Dimethylformamid und Sulfoxide, wie Diemthylsulfoxid.

Die erfindungsgemäße Umsetzung wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyltrimethyl-ammoniumhydroxid oder Dibenzyldimethylammoniumhydroxid und Tetraphenylphosphonium-hydroxid oder Methyltriphenyl-phosphoniumhydroxid.

Die Reaktionstemperaturen können beim erfindungsgemäßen Verfahren in einem größeren Bereich variiert werden. Im allgemeinen arbeitet man zwischen 0 und 150°C, vorzugsweise bei Raumtemperatur. In einzelnen Fällen ist es vorteilhaft bei der Siedetemperatur des Lösungsmittels, beispielsweise zwischen 60 und 100°C, zu arbeiten.

Bei der Durchführung des erfindungsgemäßen Verfahrens setzt man auf 1 Mol Phenethyl-triazolyl-Derivat der Formel (II) vorzugsweise 1 bis 3 Mol Halogenid der Formel (III) ein. Die Isolierung der Endprodukte der Formel (I) erfolgt in allgemein bekannter Art und Weise.

In einer bevorzugten Ausführungsform des erfindungsgemäßen Verfahrens wird die Umsetzung in einem Zweiphasensystem, wie beispielsweise wäßrige Natron- oder Kalilauge/Toluol, unter Zusatz von 0,01-1 Mol eines Phasentransferkatalysators, wie beispielsweise Ammonium- oder Phosphoniumverbindungen, durchgeführt, wobei in der organischen Phase oder an der Grenzfläche die Ethylate entstehen und mit den in der organischen Phase befindlichen Halogeniden umgesetzt werden.

Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Metallsalz-Komplexe der Verbindungen der Formel (I) können in einfacher Weise nach üblichen Verfahren erhalten werden, so z. B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie gegen den Erreger des echten Getreidemehltaus (Erysiphe graminis) und den Erreger des Braunrostes am Weizen (Puccinia recondita), ferner zur Bekämpfung des Apfelschorfes und echter Mehltaupilze an Gurken und Äpfeln sowie von Reiskrankheiten, wie Pellicularia, eingesetzt werden.

In entsprechenden Aufwandmengen zeigen die erfindungsgemäßen Wirkstoffe auch insektizide und pflanzenwachstumsregulierende Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpulver, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage: Aromaten, wie Xylol, Toulol oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie

synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffe vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

**Herstellungsbeispiele**

**Beispiel 1**

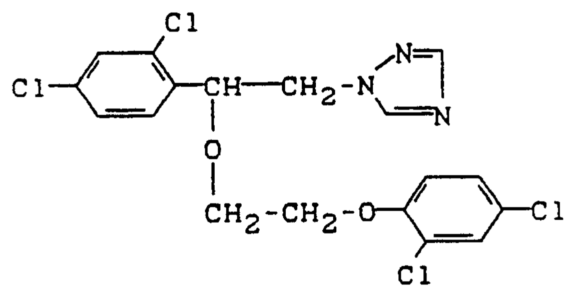

12,95 g (0,05 Mol) 1-(2,4-Dichlorphenyl)-2-(1,2,4-triazol-1-yl)-1-ethanol werden in 100 ml Toluol gelöst und mit 100 ml 40 %-iger Natronlauge sowie 1 ml Benzyldodecyldimethylammoniumchlorid versetzt. Danach läßt man 13,5 g (0,05 Mol) 2,4-Dichlorphenoxyethylbromid zutropfen und 48 Stunden bei Raumtemperatur nachrühren. Die organische Phase wird abgetrennt, dreimal mit je 100 ml gesättigter Natriumchloridlösung gewaschen, über Natriumsulfat getrocknet und eingeengt. Der Rückstand wird in 100 ml Diethylether aufgenommen, wobei er nach kurzem Stehen kristallisiert. Man erhält 7,8 g (35 % der Theorie) 1-(2,4-Dichlorphenyl)-1-(2,4-dichlorphenoxy-ethoxy)-2-(1,2,4-triazol-1-yl)-1-ethanol vom Schmelzpunkt 88 - 90°C.

**Herstellung des Ausgangsproduktes**

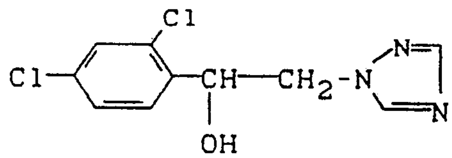

25,6 g (0,1 Mol) 1-(2-4-Dichlorphenyl)-2-(1,2,4-triazol-1-yl)-1-ethanon werden in 610 ml Methanol gelöst und bei 5 bis 10°C unter Rühren portionsweise mit 6,3 g (0,15 Mol) Natriumborhydrid versetzt. Anschließend wird eine Stunde bei Raumtemperatur gerührt und eine Stunde zum Sieden erhitzt. Nach Abdestillieren des Lösungsmittels wird der Rückstand mit 250 ml Wasser und 50 ml konzentrierter Salzsäure versetzt und 15 Minuten lang aufgekocht.

Nachdem das Reaktionsgemisch mit Natronlauge alkalisch gemacht wurde, kann das feste Reaktionsprodukt abfiltriert werden. Es wird aus wäßrigem Acetonitril umkristallisiert. Man erhält 12 g (42 % der Theorie) 1-(2-4-Dichlorphenyl)-2-(1,2,4-triazol-1-yl)-1-ethanol vom Schmelzpunkt 87°C.

In entsprechender Weise und gemäß den angegebenen Verfahren wird die in dem folgenden Beispiel angegebene Verbindung hergestellt.

**Beispiel 2**

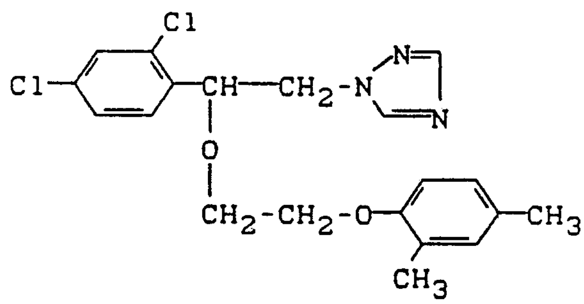

Schmelzpunkt: 72 -75°C.

**Anwendungsbeispiele**

In den nachfolgenden Anwendungsbeispielen werden die nachstehend angegebenen Verbindungen als Vergleichssubstanzen eingesetzt :

(A) 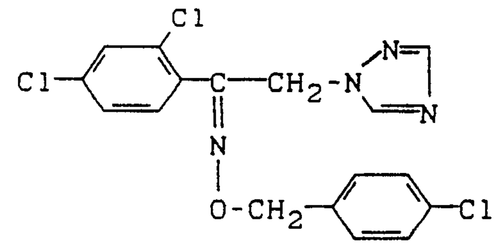 x HCl

(B) 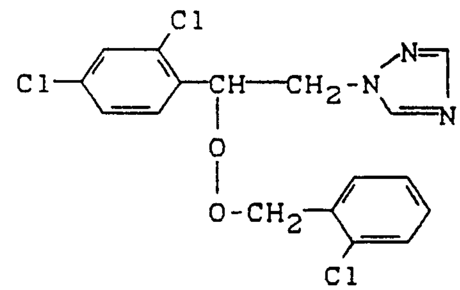

(C) 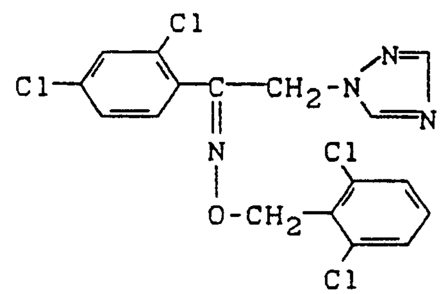 x $HNO_3$

**Beispiel A**

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt in diesem Test z. B. die Verbindung gemäß Herstellungsbeispiel 1.

**Beispiel B**

Puccinia-Test (Weizen) / protektiv
Lösungsmittel: 100 Gewichtsteile Dimethylformamid
Emulgator: 0,25 Gewichtsteile Alkylarylpolyglykolether.

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia

recondita mit in einer 0,1 %-igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 %, aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel 1.

**Patentansprüche**

1. Substituierte Phenethyl-triazolyl-Derivate der Formel

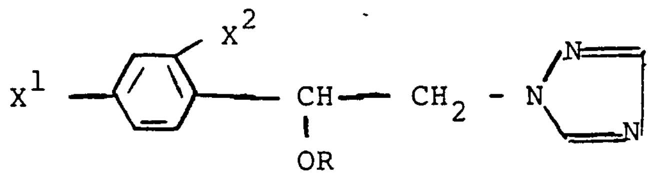

(I)

in welcher

$X^1$ und $X^2$ gleich oder verschieden sind und für Wasserstoff, Fluor, Chlor, Brom oder Halogenalkyl mit 1 oder 2 Kohlenstoffatomen und 1 bis 5 gleichen oder verschiedenen Halogenatomen stehen, wobei $X^1$ und $X^2$ aber nicht gleichzeitig Wasserstoff bedeuten, und

R für gegebenenfalls einfach bis dreifach, gleich oder verschieden im Phenylteil durch Halogen und Alkyl mit 1 bis 4 Kohlenstoffatomen substituiertes Phenoxyethyl steht,

sowie deren Säureadditions-Salze und Metallsalz-Komplexe.

2. Verbindungen der Formel (I) gemäß Anspruch 1, in denen

$X^1$ und $X^2$ gleich oder verschieden sind und für Fluor, Chlor oder Trifluormethyl stehen, sowie für Wasserstoff stehen, wenn der andere Substituent nicht Wasserstoff bedeutet, und

R für gegebenenfalls einfach bis dreifach, gleich oder verschieden im Phenylteil durch Fluor, Chlor oder Methyl substituiertes Phenoxyethyl steht.

3. Verfahren zur Herstellung von substituierten Phenethyltriazolyl-Derivaten der Formel

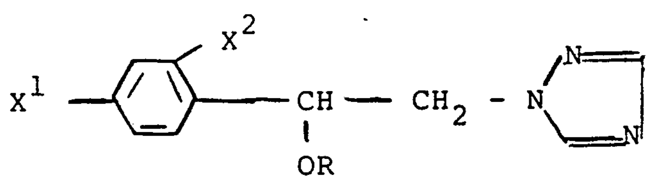

(I)

in welcher

$X^1$ und $X^2$ gleich oder verschieden sind und für Wasser-Stoff, Fluor, Chlor, Brom oder Halogenalkyl mit 1 oder 2 Kohlenstoffatomen und 1 bis 5 gleichen oder verschiedenen Halogenatomen stehen, wobei $X^1$ und $X^2$ aber nicht gleichzeitig Wasserstoff bedeuten, und

R für gegebenenfalls einfach bis dreifach, gleich oder verschieden im Phenylteil durch Halogen und Alkyl mit 1 bis 4 Kohlenstoffatomen substituiertes Phenoxyethyl steht,

sowie von deren Säureadditions-Salzen und Metallsalz-Komplexen, dadurch gekennzeichnet, daß man Phenethyltriazolyl-Derivate der Formel

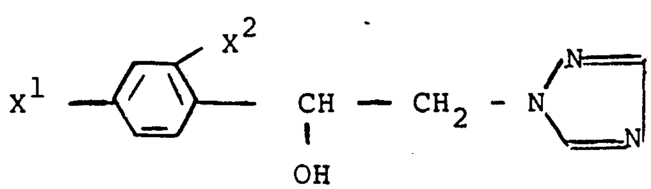

(II)

in welcher

$X^1$ und $X^2$ die oben angegebene Bedeutung haben, mit Halogeniden der Formel

Hal - R (III)

in welcher

R die oben angegebene Bedeutung hat und

Hal für Chlor oder Brom steht,

gegebenenfalls in gegenwart einer starken Base und in Gegenwart eines Verdünnungsmittels umsetzt, und gegebenenfalls noch an die so erhaltenen Verbindungen der Formel (I) eine Säure oder ein Metallsalz addiert.

4. Fungizide Mittel, gekennzeichnet durch einen Gehalt an mindestens einem substituierten Phenethyl-triazolyl-Derivat der Formel (I) gemäß Anspruch 1 bzw. an einem Säureadditions-Salz oder Metallsalz-Komplex eines substituierten Phenethyl-triazolyl-Derivates der Formel (I).

5. Verfahren zur Bekämpfung von Pilzen, dadurch gekennzeichnet, daß man substituierte Phenethyl-triazolyl-Derivate der Formel (I) gemäß Anspruch 1 bzw. deren Säureadditions-Salze oder Metallsalz-Komplexe auf Pilze oder ihren Lebensraum einwirken läßt.

6. Verwendung von substituierten Phenethyl-triazolyl-Derivaten der Formel (I) gemäß Anspruch 1 bzw. von deren Säureadditions-Salzen oder Metallsalz-Komplexen zur Bekämpfung von Pilzen.

7. Verfahren zur Herstellung von fungiziden Mitteln, dadurch gekennzeichnet, daß man substituierte Phenethyl-triazolyl-Derivate der Formel (I) gemäß Anspruch 1 oder deren Säureadditions-Salze oder Metallsalz-Komplexe mit Streckmitteln und/oder oberflächenaktiven Stoffen vermischt.

**Claims**

1. Substituted phenethyl-triazolyl derivatives of the formula

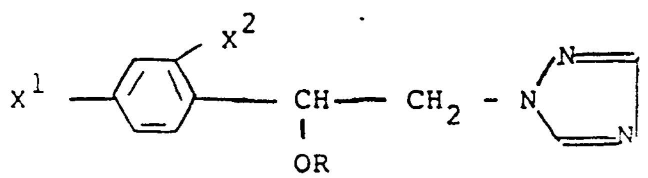
(I)

in which

$X^1$ and $X^2$ are identical or different and represent hydrogen, fluorine, chlorine, bromine or halogenoalkyl, having 1 or 2 carbon atoms and 1 to 5 identical or different halogen atoms, where $X^1$ and $X^2$ may not simultaneously denote hydrogen, and

R represents phenoxyethyl which is optionally mono-, di- or trisubstituted in the phenyl part by identical or different substituents from the group comprising halogen and alkyl having 1 to 4 carbon atoms, and acid addition salts, and metal salt complexes thereof.

2. Compounds of the formula (I) according to Claim 1, wherein

$X^1$ and $X^2$ are identical or different and represent fluorine, chlorine or trifluoromethyl; and represent hydrogen if the other substituent does not denote hydrogen, and

R represents phenoxyethyl which is optionally mono-, di- or tri-substituted in the phenyl part by identical or different substituents from the group comprising fluorine, chlorine and methyl.

3. Process for the preparation of substituted phenethyl-triazolyl derivatives of the formula

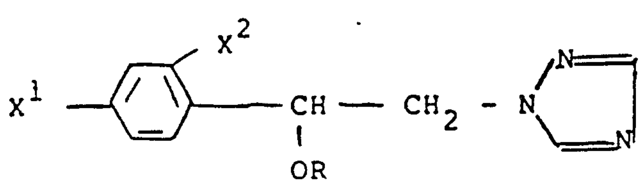
(I)

in which

$X^1$ and $X^2$ are identical or different and represent hydrogen, fluorine, chlorine, bromine or halogenoalkyl having 1 or 2 carbon atoms and 1 to 5 identical or different halogen atoms where $X^1$ and $X^2$ may not simultaneously denote hydrogen, and

R represents phenoxyethyl which is optionally mono-, di- or tri-substituted in the phenyl part by identical or different substituents from the group comprising halogen and alkyl having 1 to 4 carbon atoms, and acid addition salts and metal salt complexes thereof, characterised in that phenethyl-triazolyl derivatives of the formula

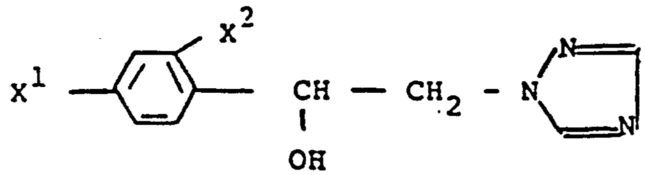

(II)

in which

$X^1$ and $X^2$ have the abovementioned meaning, are reacted with halides of the formula

Hal - R (III)

in which

R has the abovementioned meaning and

Hal represents chlorine or bromine,

if appropriate in the presence of a strong base and in the presence of a diluent, and, if appropriate, an acid or a metal salt is then added onto the compounds of the formula (I) thus obtained.

4. Fungicidal agents, characterised in that they contain at least one substituted phenethyl-triazolyl derivative of the formula (I) according to Claim 1, or an acid addition salt or metal salt complex of a substituted phenethyl-triazolyl derivative of the formula (I).

5. Method of combating fungi, characterised in that substituted phenethyl-triazolyl derivatives of the formula (I) according to Claim 1 or acid addition salts or metal salt complexes thereof are allowed to act on fungi or their environment.

6. Use of substituted phenethyl-triazolyl derivatives of the formula (I) according to Claim 1 or acid addition salts or metal salt complexes thereof for combating fungi.

7. Process for the preparation of fungicidal agents, characterised in that substituted phenethyl-triazolyl derivatives of the formula (I) according to Claim 1 or acid addition salts or metal salt complexes thereof are mixed with extenders and/or surface-active agents.

**Revendications**

1. Dérivés substitués de phénéthyl-triazolyle de formule:

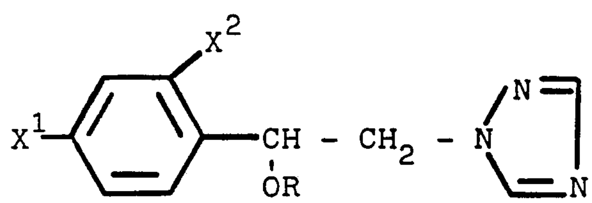

(I)

dans laquelle

$X^1$ et X2 sont identiques ou différents et représentent chacun un atome d'hydrogène, un atome de fluor, un atome de chlore, un atome de brome ou un groupe halogénalkyle contenant 1 ou 2 atomes de carbone et 1 à 5 atomes d'halogènes identiques ou différents, cependant que $X^1$ et $X^2$ ne représentent pas simultanément un atome d'hydrogène, et

R représente un groupe phénoxyéthyle éventuellement substitué une à trois fois de manière identique ou différente dans la fraction phényle par un atome d'halogène et un groupe alkyle contenant 1 à 4 atomes de carbone,

ainsi que leurs sels d'addition d'acide et leurs complexes de sels métalliques.

2. Composés de formule (I) selon la revendication 1, dans lesquels:

$X^1$ et $X^2$ sont identiques ou différents et représentent chacun un atome de fluor, un atome de chlore ou un groupe trifluorométhyle, ainsi qu'un atome d'hydrogène lorsque l'autre substituant ne représente pas un atome d'hydrogène, et

R représente un groupe phénoxyéthyle éventuellement substitué une à trois fois de manière identique ou différente dans la fraction phényle par un atome de fluor, un atome de chlore ou un groupe méthyle.

3. Procédé de préparation de dérivés substitués de phénéthyl-triazolyle de formule:

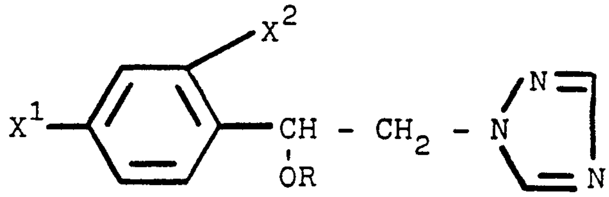

(I)

dans laquelle

$X^1$ et $X^2$ sont identiques ou différents et représentent chacun un atome d'hydrogène, un atome de fluor, un atome de chlore, un atome de brome ou un groupe halogénalkyle contenant 1 ou 2 atomes de carbone et 1 à 5 atomes d'halogènes identiques ou différents, $X^1$ et $X^2$ ne représentant pas toutefois simultanément un atome d'hydrogène, et

R représente un groupe phénoxyéthyle éventuellement substitué une à trois fois de manière identique ou différente dans la fraction phényle par un atome d'halogène et par un groupe alkyle contenant 1 à 4 atomes de carbone,

ainsi que de leurs sels d'addition d'acide et de leurs complexes de sels métalliques, caractérisé en ce qu'on fait réagir des dérivés de phénéthyl-triazolyle de formule:

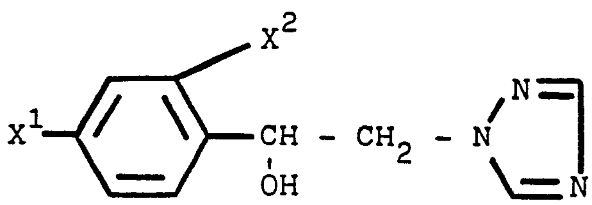

(II)

dans laquelle

$X^1$ et $X^2$ ont les significations indiquées ci-dessus, avec des halogénures de formule:

Hal -R (III)

dans laquelle

R a la signification indiquée ci-dessus, et

Hal représente un atome de chlore ou un atome de brome,

éventuellement en présence d'une base forte et en présence d'un diluant et, aux composés de formule (I) ainsi obtenus, on ajoute encore éventuellement un acide ou un sel métallique.

4. Agents fongicides, caractérisés en ce qu'ils contiennent au moins un dérivé substitué de phénéthyl-triazolyle de formule (I) selon la revendication 1 ou un sel d'addition d'acide ou encore un complexe d'un sel métallique d'un dérivé substitué de phénéthyl-triazolyle de formule (I).

5. Procédé en vue de combattre les champignons, caractérisé en ce qu'on fait agir des dérivés substitués de phénéthyl-triazolyle de formule (I) selon la revendication 1 ou leurs sels d'addition d'acide ou encore leurs complexes de sels métalliques sur des champignons ou leurs biotopes.

6. Utilisation de dérivés substitués de phénéthyl-triazolyle de formule (I) selon la revendication 1 ou de leurs sels d'addition d'acide ou encore de leurs complexes de sels métalliques en vue de combattre les champignons.

7. Procédé de préparation d'agents fongicides, caractérisé en ce qu'on mélange des dérivés substitués de phénéthyl-triazolyle de formule (I) selon la revendication 1 ou leurs sels d'addition d'acide ou encore leurs complexes de sels métalliques avec des diluants et/ou des substances tensio-actives.